# EUROPEAN PATENT SPECIFICATION

(11) **EP 3 441 107 B1**
(45) Date of publication and mention of the grant of the patent: **20.01.2021**
(21) Application number: 17779369.2
(22) Date of filing: 06.04.2017
(51) Int. Cl.: A61M 37/00, A61L 31/14, A61L 31/16, A61L 31/10, A61K 9/00, A61L 31/02, A61P 17/00, A61P 29/00, A61P 43/00

(54) **MICRONEEDLE USING BIOABSORBABLE METAL**
MIKRONADEL MIT BIORESORBIERBAREM METALL
MICRO-AIGUILLE UTILISANT UN MÉTAL BIOABSORBABLE

(30) Priority: 07.04.2016 KR 20160042690; 29.12.2016 KR 20160181865
(43) Date of publication of application: 13.02.2019
(73) Proprietor: Labnpeople Co.,Ltd., Yangju-si, Gyeonggi-do 11477 (KR)
(72) Inventor: CHO, Sung Youn, Uijeongbu-si Gyeonggi-do 11786 (KR); KIM, Jong Tack, Uijeongbu-si Gyeonggi-do 11752 (KR); CHOO, Hyun Wook, Seongnam-si Gyeonggi-do 13647 (KR)
(74) Representative: Lahrtz, Fritz
(86) International application number: PCT/KR2017/003775
(87) International publication number: WO 2017/176069

(56) References cited:
- WO-A1-2005/016441
- WO-A2-2013/192083
- KR-A- 20100 025 509
- US-A1- 2015 157 840
- US-B2- 8 778 012

## Description

### Technical Field

The present invention relates generally to a microneedle using a bioabsorbable metal according to the preamble of claim 1, and, more particularly, to a microneedle using a bioabsorbable metal, the microneedle being capable of supplying a drug into the body together with functional substances beneficial to the human body.

### Background Art

As well known in the art, a drug delivery system (DDS) refers to a technology for delivering drugs having pharmacological activity to cells, tissues, organs, and the like using various physical and chemical methods.

Among all drug delivery systems, oral drug delivery, which provides delivery of a drug through the mouth, is the most preferred route of drug administration, and other routes of drug administration include transdermal drug delivery which provides delivery of a drug through the skin to a desired location in the human body, and the like. Of these, drug delivery in which a metal hypodermic needle is used to be pierced through the skin to create a hole that serves as conduit for delivery of a liquid drug, that is, drug delivery using a syringe, has been widely used for a long time.

However, drug delivery using a syringe is problematic in that patients may suffer pain upon drug injection, and repeated vaccination may cause inconvenience. Additionally, reuse of the needle due to lack of management of the syringe may cause infection to patients.

Moreover, the above method requires a vaccination provider who has knowledge of the use of a syringe, so a ) patient may not administer a drug using a syringe by himself or herself.

Accordingly, in recent years, a micro-scaled transdermal microneedle which is much smaller than a pen-type syringe has been developed and used for improving drug delivery using a syringe.

A micro needle is a system for physically piercing the stratum corneum of the skin and delivering a drug. In 1998, the Prausnitz group at Georgia Institute of Technology developed a microneedle array with a silicon device using a semiconductor process technology and proposed a possibility of drug delivery. Accordingly, much research regarding the microneedle has been actively carried out, and microneedles of various sizes and shapes are being made based on various materials such as metals, polymers, glasses, ceramics, and the like as well as silicon.

Additionally, microneedles are used for delivery of active substances, such as drugs, vaccines, and the like in vivo, and detection and biopsy of analytes in the body. The microneddles also find application in injecting skin-care substances or drugs into skin tissues or extracting body fluids such as blood under the skin. Thus, microneedles are one of the drug delivery systems that have been rapidly used in various fields in recent years because local and sustained drug injection is possible and pain on insertion into the skin can be minimized.

However, a microneedle in the related art is required to be in contact with the skin of the user for a long time in order to spread a drug contained on the microneedle into the body, thus causing inflammation to the skin of the user due to material properties of the needle.

Additionally, the microneedle has to maintain a sufficiently high physical strength such that the needle can be inserted into the skin without bending or breaking and thus is required to have properties for controlling the strength and shape suitable therefor.

However, the microneedle in the related art is generally made of a degradable polymer and a metallic material. Accordingly, a microneedle made of a degradable polymer may not be sufficiently effective in delivering a drug into the body due to low strength inherent to the degradable polymer and difficulty of molding. Furthermore, a microneedle made of a metallic material may cause an inflammatory response to a user when the needle is broken when inserted in the subcutaneous tissue of the skin, and should not be used for a user who is allergic to the metallic material. Additionally, the microneedle made of a metallic material has a limit in manufacturing.

Thus, the applicant of the present invention has proposed the present invention in order to solve the above-mentioned problems. The related art document related thereto corresponds to Korean Patent No. 10-1634911, entitled "Method of manufacturing a micro-sized needle".

As further related art documents which were also not able to solve the underlying problems of the present invention, the following documents are known:

First of all, WO 2005/016441 A1 discloses a microprojection array having at least first and second microprojections, wherein the first and second microprojections have inner and outer faces, with the first microprojection inner face being disposed substantially parallel to the second microprojection inner face. Also, a biocompatible coating is disposed on the first and second microprojection inner faces, with the first and second ) microprojections being adapted to substantially restrict contact of the coating with biological tissue during insertion of the first and second microprojections into the tissue.

Next, US 8,778,012 B2 teaches an extracellular matrix construct with a biodegradable support scaffold that includes a plurality of biodegradable microneedles capable of piercing tissue and anchoring therein, and with at least a first layer of first ECM material disposed on the top surface of the support scaffold.

As additional document, WO 2013/192083 A2 shows and described a 3D microelectrode device including a flexible substrate containing poly-dimethyl siloxane (PDMS). Here, the device may be fabricated in a miniature form factor suitable for attachment to a small organ such as a lateral gastrocnemius muscle of a live rat. In addition to providing a miniaturized, conformable attachment, the device provides an anchoring action via one or more microelectrodes, each having an insertable tip particularly shaped to provide the anchoring action. Furthermore, a base portion of each of the microelectrodes is embedded inside conductive poly-dimethyl siloxane cPDMS. The cPDMS is contained in a pad that is coupled to a conductive track embedded in the flexible substrate.

Moreover, KR 2010 0025509 discloses a microneedle device having microneedles that are easy to inject into the skin surface and dissolve or swell in the skin surface, wherein the microneedle device has a base and a microneedle for insertion into the skin surface of a conical or polygonal cone arranged on the base, which microneedle is formed from a material which contains at least 50% by weight of one or more biomaterials selected from the group consisting of chitosan, collagen, gelatin and hyaluronic acid and which can be dissolved or swelled in vivo.

Finally, US 2015/157840 A1 shows and describes a microneedle sheet comprising a plurality of microneedles formed on a sheet substantially along a principal surface of the sheet, wherein the sheet is bent to raise the microneedles from the principal surface, and wherein the raised microneedles pierce skin.

### Disclosure

### Technical Problem

Accordingly, the present invention has been made keeping in mind the above problems occurring in the related art, and an objective of the present invention is to provide a microneedle, which is configured to prevent pain and infection from occurring to a user even when the microneedle is in contact with the skin of the user for a long time or remains in the body.

Another objective of the present invention is to provide a microneedle, which is capable of delivering a drug carried on the microneedle into the body of a user together with minerals beneficial to the human body.

### Technical Solution

In order to accomplish the above objectives, the present invention provides a microneedle using a bioabsorbable metal having the features of claim 1, the microneedle including: a substrate portion; a needle portion provided at the substrate portion and protruding from the substrate portion to be inserted into the skin; and a perforated portion provided at a junction between the needle portion and the substrate portion, wherein either of the substrate portion and the needle portion is made of the bioabsorbable metal.

When the substrate portion is removed from the skin, the needle portion may be separated from the substrate portion and thus remain in the skin.

The needle portion may include: a needle cutout formed in the substrate portion; a needle first portion connected at a first end thereof to the substrate portion while being received in the needle cutout; and a needle second portion received in the needle cutout while being connected at a first end thereof with a second end of the needle first portion.

The needle second portion may be formed in a shape such that a width thereof gradually decreases from the first end to a second end thereof, the first end being larger than the needle first portion in width so as to have a barb formed thereat.

When the substrate portion which has been in contact with the skin is removed from the skin, the barb may become caught in a subcutaneous tissue such that the needle first portion and the needle second portion are embedded in the subcutaneous tissue.

The perforated portion may include multiple perforations provided at the first end of the needle first portion to be arranged along a transverse direction of the needle first portion at predetermined intervals.

The perforated portion may further include a notch, wherein the notch is formed at the first end of the needle first portion to be positioned at opposite corners of the needle first portion in a width direction thereof, respective notches communicating with the needle cutout.

Either of the needle first portion and the needle second portion may be provided with multiple drug carrying ) recesses formed on a peripheral surface thereof to be arranged along the peripheral surface of either of the needle first portion and the needle second portion.

The drug carrying recesses may be further provided on either of an upper surface and a lower surface 5 of either of the needle first portion and the needle second portion.

The bioabsorbable metal may include at least one of magnesium, calcium, zinc, and iron.

The microneedle using the bioabsorbable metal according to the embodiment of the present invention having the above described configuration can deliver substances (magnesium, calcium, zinc, iron, and the like) included in the bioabsorbable metal into the body as well as delivering drugs for treatment and thus supply minerals together with the drugs to the user.

Furthermore, the microneedle using the bioabsorbable metal according to the embodiment of the present invention can allow the needle portion to remain in the subcutaneous tissue through a simple process of removing the substrate portion in contact with the skin therefrom. This makes it possible for the bioabsorbable metal beneficial to the human body to efficiently remain in the body of the user. Thus, the body of the user can accommodate the beneficial bioabsorbable metal without undergoing any other application or treatment.

Furthermore, the microneedle using the bioabsorbable metal according to the embodiment of the present invention can allow the by-products (hydrogen gas), which are produced when the needle portion made of the bioabsorbable metal degrades in the subcutaneous tissue, to provide a swelling effect in the subcutaneous tissue and thus reduce the skin wrinkles of the user. For example, the bioabsorbable metal constituted by the magnesium produces magnesium ions, magnesium hydroxide, and hydrogen gas as the by-products, resulting in the magnesium being intensively injected into a local region of the skin and resulting in the produced hydrogen gas swelling in the subcutaneous tissue to reduce skin wrinkles of the user. Accordingly, approximately 1 L of hydrogen gas is produced per 1 g of magnesium and thus the present invention can be highly efficient even in a small amount.

Furthermore, the microneedle using the bioabsorbable metal according to the embodiment of the present invention can allow the substrate portion and the needle portion that are made of the bioabsorbable metal to serve as a drug delivery enhancer and thus to provide rapid delivery of a drug carried thereon to the subcutaneous tissue. This makes it possible for a required drug to be quickly delivered into the body of the user for treatment.

### Description of Drawings

FIG. 1 is a perspective view showing a microneedle according to an embodiment of the present invention.
FIG. 2 is an enlarged plan view showing a portion A shown in FIG. 1.
FIG. 3 is a perspective view showing a state in which a needle portion is erected on a substrate portion according to the embodiment of the present invention.
FIG. 4 is an enlarged perspective view showing a portion A shown in FIG. 3.
FIG. 5 is a plan view showing perforations formed in the needle portion according to the embodiment of the present invention.
FIG. 6 is a view showing the needle portion remaining in skin when the substrate portion attached to the skin is removed from the skin.

### Mode for Invention

Advantages and features of the present invention and methods of accomplishing the same may be understood more readily by reference to the following detailed description of exemplary embodiments and the accompanying drawings.

Advantages and features of the present invention and methods of accomplishing the same may be understood more readily by reference to the following detailed description of exemplary embodiments and the accompanying drawings. The present invention may, however, be embodied in many different forms and should not be construed as being limited to the embodiments set forth herein. Rather, these embodiments are provided so that this disclosure will be thorough and complete and will fully convey the concept of the invention to those skilled in the art, and the present invention will only be defined by the appended claims.

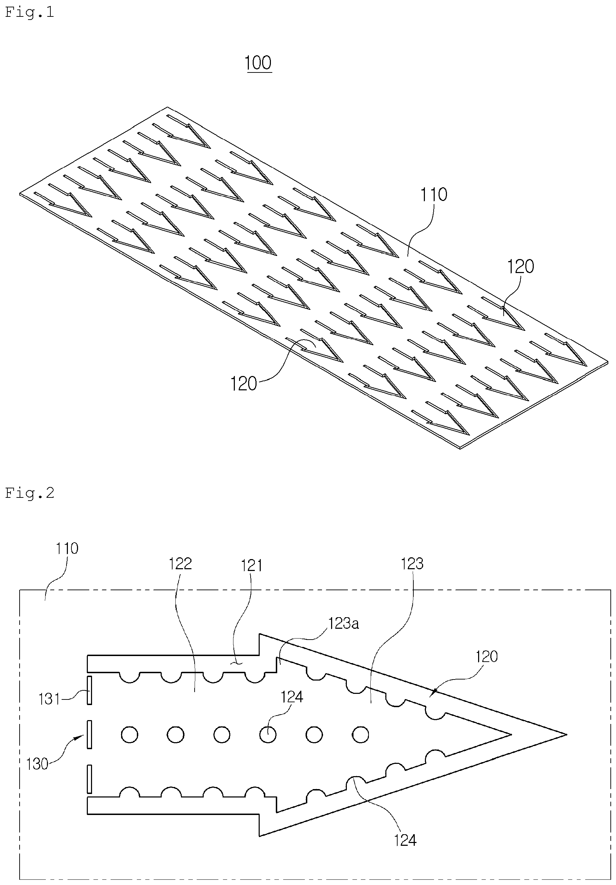

Hereinafter, a microneedle using a bioabsorbable metal according to an embodiment of the present invention will be described in detail with reference to FIGS. 1 to 6. FIG. In the following description, it is to be noted that, when the functions of conventional elements and the detailed description of elements related with the present invention may make the gist of the present invention unclear, a detailed description of those elements will be omitted.

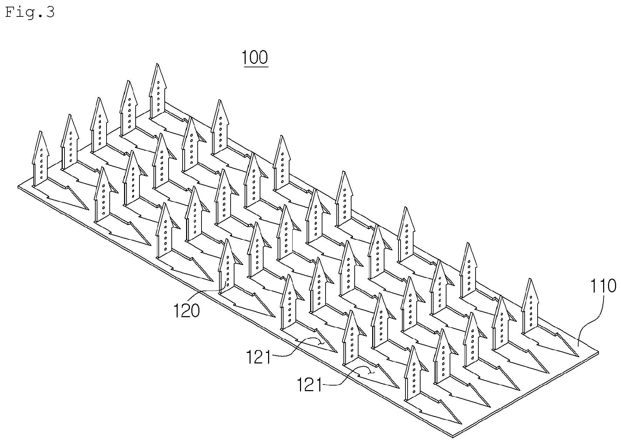

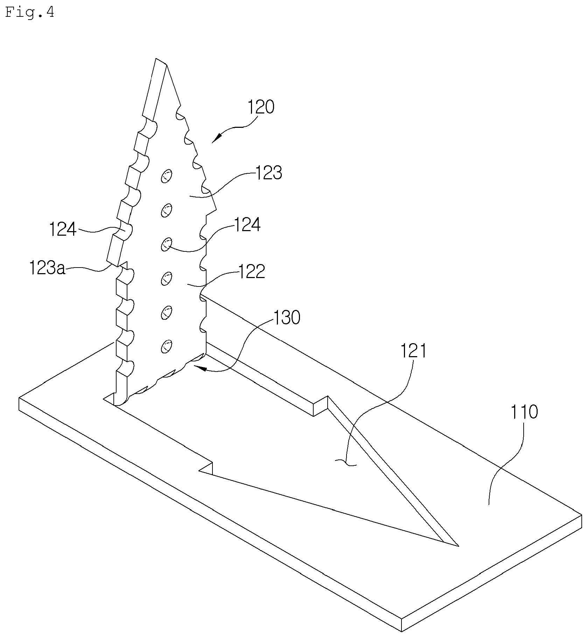

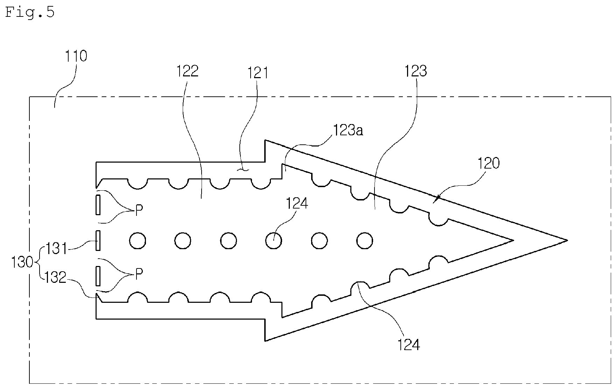

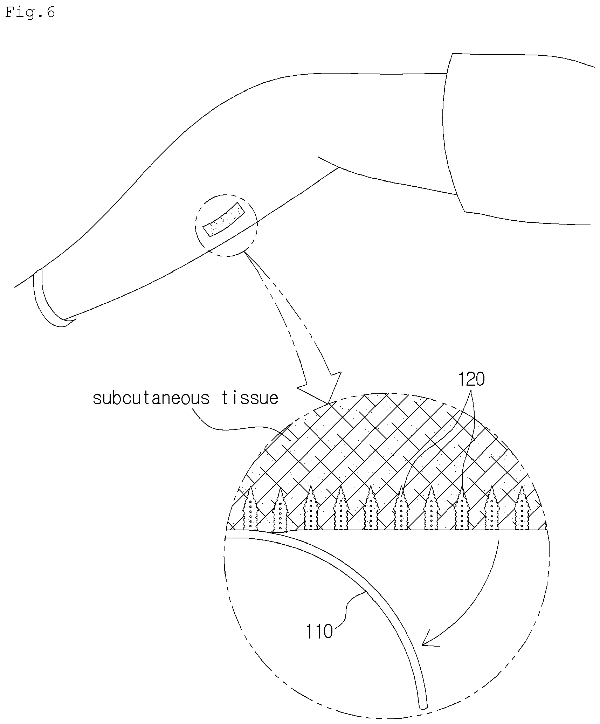

FIG. 1 is a perspective view showing a microneedle according to an embodiment of the present invention, FIG. 2 is an enlarged plan view showing a portion A shown in FIG. 1, FIG. 3 is a perspective view showing a state in which a needle portion is erected on a substrate portion according to the embodiment of the present invention, FIG. 4 is an enlarged perspective view showing a portion A shown in FIG. 3, FIG. 5 is a plan view showing perforations formed in the needle portion according to the embodiment of the present invention, and FIG. 6 is a view showing the needle portion remaining in skin when the substrate portion attached to the skin is removed from the skin.

As shown in FIGS. 1 to 6, a microneedle 100 using a bioabsorbable metal according to an embodiment of the ) present invention includes: a substrate portion 110 being in contact with skin; a needle portion 120 provided at the substrate portion 110 and protruding from the substrate portion 110 to be inserted into the skin; and a perforated portion 130 provided at a junction between the needle 5 portion 120 and the substrate portion 110.

The substrate portion 110 may have a thin plate shape having a predetermined area and thickness, and may carry a drug to be delivered to the subcutaneous tissue of the skin.

As a method of carrying a drug on the substrate portion 110, there are various known methods such as a method of coating the substrate portion 110 by immersing the substrate portion in a container containing the drug therein, a method of coating the substrate portion 110 by applying the drug to the substrate portion, and the like.

For reference, drugs carried on the substrate portion 110 may include drugs or genetic materials for disease prevention and treatment and epidermal growth factor (EGF) or hyaluronic acid for skin care.

Meanwhile, the substrate portion 110 may be made of the bioabsorbable metal. In other words, the substrate portion 110 may be made of a metal including at least one of magnesium, calcium, zinc, and iron, which is used as the bioabsorbable metal.

Additionally, the substrate portion 110 having the above-described configuration may be attached to the skin of a user in the form of a patch in a state of being placed on an adhesive sheet (not shown) coated with an adhesive material.

The needle portion 120 is a component that can be formed by processing the substrate portion 110 with a laser cutting device, and may be provided in plural such that multiple needle portions are arranged on the substrate portion 110 at predetermined intervals. Accordingly, the needle portion 120 may also be made of the bioabsorbable metal.

Furthermore, as shown in FIGS. 3 and 4, the needle portion 120 may be bent to protrude vertically on the substrate portion 110 through a known forming process. In other words, the needle portion 120 can be said to be a portion that is inserted into the subcutaneous tissue of the skin of the user to deliver a drug when the substrate portion 110 comes into contact with the skin of the user.

As shown in FIG. 2, the needle portion 120 may include a needle cutout 121 formed in the substrate portion 110, a needle first portion 122 connected at a first end thereof to the substrate portion 110 while being received in the needle cutout 121, and a needle second portion 123 received in the needle cutout 121 while being connected at a first end thereof with a second end of the needle first portion 122.

The needle cutout 121 may be formed on the substrate portion 110 by cutting using a laser cutting machine or by sheet metal working. In this process, the needle first portion 122 and the needle second portion 123 may be formed.

In other words, the shape and size of the needle cutout 121 may be a factor that determines the shape and size of the needle first portion 122 and the needle second portion 123. The shape and size of the needle cutout may vary corresponding to the shape and size of the needle first portion 122 and the needle second portion 123.

The needle first portion 122 and the needle second portion 123 may substantially have an arrowhead shape. The needle first portion 122 may have a predetermined width and may be connected at the first end thereof in a lengthwise direction thereof to an inner edge of the substrate portion 110, the inner edge defining the needle cutout 121.

Furthermore, the needle second portion 123 may be formed in an arrowhead shape such that a width thereof gradually decreases from the first end to a second end thereof in a lengthwise direction thereof. The first end of the needle second portion in the lengthwise direction thereof may be connected with the second end of the needle first portion 122 in the lengthwise direction thereof as described above.

Additionally, the needle second portion 123 may be configured such that the first end thereof in the lengthwise direction thereof is larger than the second end of the needle first portion 122 in the lengthwise direction thereof in width so as to have a barb 123a formed at opposite corners of the needle second portion in a width direction thereof.

The perforated portion 130 may be configured such that when the substrate portion 110 which has been in contact with the skin is removed therefrom, the first end of the needle first portion 122 in the lengthwise direction thereof is efficiently separated from the substrate portion 110, resulting in the needle first and second portions 122 and 123 inserted into the subcutaneous tissue of the skin remaining therein. In other words, as shown in FIG. 6, when the substrate portion 110 is removed from the skin, the perforated portion 130 may allow the needle portion 120 to remain in the subcutaneous tissue of the skin.

As shown in FIG. 2, the perforated portion 130 may be configured as multiple perforations 131 provided at the first end of the needle first portion 122 in the lengthwise direction thereof to be arranged along a width direction of the needle first portion 122 at predetermined intervals.

The perforations 131 are perforated in a thickness direction of the substrate portion 110 and serve to reduce the area of a junction between the needle first ) portion 122 and the substrate portion 110 such that the first end of the needle first portion in the lengthwise direction thereof is efficiently separated from the substrate portion.

Hereinafter, a process in which the needle first portion 122 and the needle second portion 123 are caused to remain in the subcutaneous tissue of the skin due to the perforated portion 130 configured as described above will be described in more detail as follows.

The needle first portion 122 and the needle ) second portion 123 inserted into the subcutaneous tissue of the skin may be embedded in the subcutaneous tissue by retraction of the subcutaneous tissue. Herein, when the substrate portion 110 is removed from the skin by the user and thus the needle first portion 122 and the needle second portion 123 are pulled with a force larger than the retraction of the subcutaneous tissue, the needle first portion 122 and the needle second portion 123 are caused to be pulled out of the skin and separated together with the substrate portion 110.

In the embodiment of the present invention, however, the multiple perforations 131 of the perforated portion 130 are formed at the junction between the substrate portion 110 and the needle first portion 122, thereby causing the force of the substrate portion 110 acting to pull the needle first portion 122 and the needle second portion 123 to decrease to be smaller than the retraction provided by the subcutaneous tissue. Because of this, the needle first portion 122 and the needle second portion 123 remain in the subcutaneous tissue.

Furthermore, the barb 123a formed at the needle second portion 123 may become caught in the subcutaneous tissue, resulting in the needle first portion 122 and the needle second portion 123 inserted into the subcutaneous tissue being more firmly embedded therein. Thus, when the substrate portion 110, which has been in contact with the skin, is removed therefrom, the junction between the needle first portion 122 and the substrate portion 110 is caused to be efficiently broken.

Herein, the needle first portion 122 and the needle second portion 123 remaining in the subcutaneous tissue of the skin may deliver the drug carried on the substrate portion 110 to the subcutaneous tissue together with minerals contained in the bioabsorbable metal. In other words, the magnesium, calcium, zinc, and iron used as the bioabsorbable metal may be delivered to the subcutaneous tissue, resulting in minerals contained therein being supplied into the body.

For reference, the bioabsorbable metal has been manufactured as a magnesium-based alloy for application to orthopedic implants and has been commercialized domestically and overseas. The bioabsorbable metal applied to orthopedic implants has focused on reducing the degradation rate in the body as much as possible or improving the corrosion resistance for the purpose of safe fracture fixation.

However, the bioabsorbable metal used for the microneedle 100 according to the embodiment of the present invention accelerates the degradation rate thereof in the body, unlike the bioabsorbable metal used for orthopedic implants. Thus, a mechanism of supplying minerals as well as releasing the drug may be applied.

For example, magnesium, calcium, and zinc, which are used as the bioabsorbable metal, have a mechanism of releasing hydrogen gas through a reaction with water while degrading as shown in the following chemical formulas 1 to 3, respectively.

[Chemical formula 1] Mg+2H₂O→Mg (OH)₂+H₂ (gas)

[Chemical formula 2] Ca+2H₂O→Ca (OH)₂+H₂ (gas)

[Chemical formula 3] Zn+2H₂O→Zn (OH)₂+H₂ (gas)

The substrate portion 110 and the needle portion 120 made of the bioabsorbable metal described above release ions and degradation products from the subcutaneous tissue during degradation, resulting in production of hydrogen gas as by-products. The hydrogen gas exerts a swelling effect in the subcutaneous tissue, leading to an anti-wrinkle effect in the skin.

Additionally, ZnO and MgCl, which are byproducts produced when magnesium and zinc constituting the bioabsorbable metal are inserted into the body, may be caused to remain in the subcutaneous tissue of the skin so as to serve as a drug delivery enhancer, the drug delivery enhancer improving absorption of the drug carried on the substrate portion 110 and the needle portion 120 to the subcutaneous tissue. Thus, the substrate portion 110 and the needle portion 120 made of the bioabsorbable metal enables the drug carried thereon to be effectively delivered to the user.

Meanwhile, the perforated portion 130 may further include a notch 132 as shown in FIG. 5. The notch 132 may be formed at the first end of the needle first portion 122 in the lengthwise direction thereof to be positioned at opposite corners of the needle first portion 122 in a width direction thereof, respective notches communicating with the needle cutout 121.

The notches 132 serve to enable the first end of the needle first portion 122 in the lengthwise direction thereof to be more efficiently separated from the substrate portion 110 when the substrate portion 110 which has been in contact with the skin is removed therefrom and also serve to relieve stretching of the skin of the user which may be caused when the needle first portion 122 is separated from the substrate portion 110.

More specifically explained, the notches 132 may be formed such that when the first end of the needle first portion 122 in the lengthwise direction thereof connected to the substrate portion 110 is separated from the substrate portion 110, either of the opposite corners of the needle first portion in the width direction thereof is primarily broken. This causes the needle first portion 122 to be efficiently separated.

In other words, the notches 132 enable that the junction P between the substrate portion 110 and the needle first portion 122 to be broken along the width direction of the needle first portion 122, the junction having the multiple perforations 131 arranged at intervals. This causes the needle first portion 122 to be quickly separated from the substrate portion 110. Additionally, the notches 131 relive the stretching of the skin of the user which may be caused when the needle portion 120 which has been in contact with the skin is removed therefrom.

Furthermore, either of the needle first portion 122 and the needle second portion 123 may have a drug carrying recess 124 formed on a peripheral surface thereof.

Multiple drug carrying recesses 124 may be provided along the peripheral surface of either of the needle first portion 122 and the needle second portion 123 at predetermined intervals to form a serrated shape. Each of the drug carrying recesses 124 may be formed to correspond to the thickness of either of the needle first portion 122 and the needle second portion 123.

The drug carrying recesses 124 serve to control the amount of the drug to be carried on either of the needle first portion 122 and the needle second portion 123. In other words, the drug carrying recesses 124 allows the side surface area of either of the needle first portion 122 and the needle second portion 123 to increase or decrease according to the number of the recesses. Accordingly, controlling of the amount of the drug to be carried on either of the needle first portion 122 and the needle second portion 123 is possible.

Additionally, the drug carrying recesses 124 are formed on the peripheral surface of either of the needle first portion 122 or the needle second portion 123 to have a serrated shape as described above, resulting in the subcutaneous tissue being retracted in a state of being received in spaces defined by the drug carrying recesses 124.

Thus, the drug carrying recesses 124 formed on the peripheral surface of either of the needle first portion 122 or the needle second portion 123 enable that the needle first portion 122 and the needle second portion 123 inserted into the subcutaneous tissue are more firmly embedded in the subcutaneous tissue. This makes it possible to prevent the needle first portion 122 from being pulled out of the skin by the substrate portion 110 and thus separated from the subcutaneous tissue when the substrate portion 110 which has been in contact with the skin is removed therefrom.

For reference, the multiple drug carrying recesses 124 are not limited to the peripheral surface of either of the needle first portion 122 and the needle second portion 123 but may be provided on either of an upper surface and a lower surface of either of the needle first portion 122 and the needle second portion 123 to be arranged at predetermined intervals. Accordingly, controlling of the amount of the drug to be carried on either of the upper and lower surfaces of either of the needle first portion 122 and the needle second portion 123 is also possible.

The microneedle 100 using the bioabsorbable metal according to the embodiment of the present invention having the above described configuration can deliver substances (magnesium, calcium, zinc, iron, and the like) included in the bioabsorbable metal into the body as well as delivering drugs for treatment and thus supply minerals together with the drugs to the user.

Furthermore, the microneedle 100 using the bioabsorbable metal according to the embodiment of the present invention can allow the needle portion 120 to remain in the subcutaneous tissue through a simple process of removing the substrate portion 110 in contact with the skin therefrom. This makes it possible for the bioabsorbable metal beneficial to the human body to efficiently remain in the body of the user. Thus, the body of the user can accommodate the bioabsorbable metal beneficial to the human body without undergoing any other application or treatment.

Furthermore, the microneedle 100 using the bioabsorbable metal according to the embodiment of the present invention can allow the by-products (hydrogen gas), which are produced when the needle portion 120 made of the bioabsorbable metal degrades in the subcutaneous tissue, to provide the swelling effect in the subcutaneous tissue and thus reduce the skin wrinkles of the user.

Furthermore, the microneedle 100 using the bioabsorbable metal according to the embodiment of the present invention can allow the substrate portion 110 and the needle portion 120 that are made of the bioabsorbable metal to serve as the drug delivery enhancer and thus to provide rapid delivery of the drug carried thereon to the subcutaneous tissue. This makes it possible for a required drug to be quickly delivered into the body of the user for treatment.

Although a preferred embodiment of the present invention has been described for illustrative purposes, those skilled in the art will appreciate that various modifications, additions and substitutions are possible, without departing from the scope of the accompanying claims.

Therefore, the scope of the present invention is defined by the accompanying claims rather than the description which is presented above. Moreover, the present invention is intended to cover not only the exemplary embodiments, but also various alternatives, modifications, equivalents and other embodiments that may be included within the scope of the appended claims.

### Industrial Applicability

The microneedle using the bioabsorbable metal according to the present invention can be commercialized and used in various industrial fields such as medical field, skin beauty field, and the like.

## Claims

1. A microneedle (100) using a bioabsorbable metal, the microneedle (100) comprising:
a substrate portion (110); and
a needle portion (120) provided at the substrate portion (110) and protruding from the substrate portion (110) to be inserted into skin;
**characterized in that**
a perforated portion (130) is provided at a junction between the needle portion (120) and the substrate portion (110),
wherein either of the substrate portion (110) and the needle portion (120) is made of the bioabsorbable metal.

2. The microneedle (100) of claim 1, wherein when the substrate portion (110) is removed from the skin, the needle portion (120) is separated from the substrate portion (110) and thus remains in the skin.

3. The microneedle (100) of claim 2, wherein the needle portion (120) includes:
a needle cutout (121) formed in the substrate portion (110);
a needle first portion (122) connected at a first end thereof to the substrate portion (110) while being received in the needle cutout (121); and
a needle second portion (123) received in the needle cutout (121) while being connected at a first end thereof with a second end of the needle first portion (122).

4. The microneedle (100) of claim 3, wherein the needle second portion (123) is formed in a shape such that a width thereof gradually decreases from the first end to a second end thereof, the first end being larger than the needle first portion (122) in width so as to have a barb (123a) formed thereat.

5. The microneedle (100) of claim 4, wherein when the substrate portion (110) which has been in contact with the skin is removed from the skin, the barb (123a) becomes caught in subcutaneous tissue such that the first and needle second portions (122, 123) are embedded in the subcutaneous tissue.

6. The microneedle (100) of claim 3, wherein the perforated portion (130) includes:
multiple perforations (131) provided at the first end of the needle first portion (122) to be arranged along a transverse direction of the needle first portion (122) at predetermined intervals.

7. The microneedle (100) of claim 6, wherein the perforated portion (130) further includes:
a notch (132), wherein
the notch (132) is formed at the first end of the needle first portion (122) to be positioned at opposite corners of the needle first portion (122) in a width direction thereof, respective notches (132) communicating with the needle cutout (121).

8. The microneedle (100) of claim 3, wherein either of the needle first portion (122) and the needle second portion (123) is provided with multiple drug carrying recesses (124) formed on a peripheral surface thereof to be arranged along the peripheral surface of either of the needle first portion (122) and the needle second portion (123).

9. The microneedle (100) of claim 8, wherein the drug carrying recesses (124) are further provided on either of an upper surface and a lower surface of either of the needle first portion (122) and the needle second portion (123).

10. The microneedle (100) of any one of claims 1 to 9, wherein the bioabsorbable metal includes at least one of magnesium, calcium, zinc, and iron.

## Patentansprüche

1. Bioabsorbierbares Metall verwendende Mikronadel (100) mit:
einem Substratabschnitt (110); und
einem Nadelabschnitt (120), der an dem Substratabschnitt (110) vorgesehen ist und von dem Substratabschnitt (110) hervorsteht, um in die Haut eingeführt zu werden;
**dadurch gekennzeichnet, dass**
ein perforierter Abschnitt (130) an einer Verbindungsstelle zwischen dem Nadelabschnitt (120) und dem Substratabschnitt (110) vorgesehen ist,
wobei entweder der Substratabschnitt (110) oder der Nadelabschnitt (120) aus dem bioabsorbierbaren Metall hergestellt ist.

2. Mikronadel (100) gemäß Anspruch 1, wobei dann, wenn der Substratabschnitt (110) von der Haut entfernt wird, der Nadelabschnitt (120) vom Substratabschnitt (110) getrennt wird und somit in der Haut verbleibt.

3. Mikronadel (100) gemäß Anspruch 2, wobei der Nadelabschnitt (120) Folgendes umfasst:
einen Nadelausschnitt (121), der in dem Substratabschnitt (110) ausgebildet ist;
einen Nadelerstabschnitt (122), der an dessen ersten Ende mit dem Substratabschnitt (110) verbunden ist, während er in dem Nadelausschnitt (121) aufgenommen ist; und
einen Nadelzweitabschnitt (123), der in dem Nadelausschnitt (121) aufgenommen ist, während er an dessen ersten Ende mit einem zweiten Ende des Nadelerstabschnitts (122) verbunden ist.

4. Mikronadel (100) gemäß Anspruch 3, wobei der Nadelzweitabschnitt (123in einer Form so ausgebildet ist, dass dessen Breite von seinem ersten Ende zu seinem zweiten Ende hin allmählich abnimmt, wobei das erste Ende breiter ist als der Nadelerstabschnitt (122), so dass an ihm ein Widerhaken (123a) ausgebildet ist.

5. Mikronadel (100) gemäß Anspruch 4, wobei dann, wenn der Substratabschnitt (110), der mit der Haut in Kontakt war, von der Haut entfernt wird, der Widerhaken (123a) sich im subkutanen Gewebe verfängt, so dass der Nadelerstabschnitt und der Nadelzweitabschnitt (122, 123) in das subkutane Gewebe eingebettet werden.

6. Mikronadel (100) gemäß Anspruch 3, wobei der perforierte Abschnitt (130) Folgendes umfasst:
mehrere Perforationen (131), die am ersten Ende des Nadelerstabschnitts (122) vorgesehen sind, um entlang einer Querrichtung des Nadelerstabschnitts (122) in vorbestimmten Intervallen angeordnet zu werden.

7. Mikronadel (100) gemäß Anspruch 6, wobei der perforierte Abschnitt (130) ferner Folgendes umfasst:
eine Ausklinkung (132), wobei
die Ausklinkung (132) an dem ersten Ende des Nadelerstabschnitts (122) ausgebildet ist, um an gegenüberliegenden Ecken des Nadelerstabschnitts (122) in dessen Breitenrichtung positioniert zu sein, wobei jeweilige Ausklinkungen (132) mit dem Nadelausschnitt (121) in Verbindung stehen.

8. Mikronadel (100) gemäß Anspruch 3, wobei entweder der Nadelerstabschnitt (122) oder der Nadelzweitabschnitt (123) mit mehreren, auf deren Umfangsfläche ausgebildeten, medikamententragenden Aussparungen (124) versehen ist, die entlang der Umfangsfläche entweder des Nadelerstabschnitts (122) oder des Nadelzweitabschnitts (123) anzuordnen sind.

9. Mikronadel (100) gemäß Anspruch 8, wobei die medikamententragenden Aussparungen (124) ferner entweder auf einer oberen oder einer unteren Fläche des Nadelerstabschnitts (122) oder des Nadelzweitabschnitts (123) vorgesehen sind.

10. Mikronadel (100) nach einem der Ansprüche 1 bis 9, wobei das bioabsorbierbare Metall mindestens eines von Magnesium, Kalzium, Zink und Eisen aufweist.

## Revendications

1. Micro-aiguille (100) utilisant un métal bioabsorbable, la micro-aiguille (100) comprenant :
une partie de substrat (110) ; et
une partie d'aiguille (120) prévue au niveau de la partie de substrat (110) et faisant saillie de la partie de substrat (110) pour être insérée dans la peau ;
**caractérisée en ce que** :
une partie perforée (130) est prévue au niveau d'une jonction entre la partie d'aiguille (120) et la partie de substrat (110),
dans laquelle l'une ou l'autre parmi la partie de substrat (110) et la partie d'aiguille (120) est réalisée à partir d'un métal bioabsorbable.

2. Micro-aiguille (100) selon la revendication 1, dans laquelle lorsque la partie de substrat (110) est retirée de la peau, la partie d'aiguille (120) est séparée de la partie de substrat (110) et reste ainsi dans la peau.

3. Micro-aiguille (100) selon la revendication 2, dans laquelle la partie d'aiguille (120) comprend :
une découpe d'aiguille (121) formée dans la partie de substrat (110) ;
une première partie d'aiguille (122) raccordée, au niveau de sa première extrémité, à la partie de substrat (110) tout en étant reçue dans la découpe d'aiguille (121) ; et
une seconde partie d'aiguille (123) reçue dans la découpe d'aiguille (121) tout en étant raccordée, au niveau de sa première extrémité, avec une seconde extrémité de la première partie d'aiguille (122).

4. Micro-aiguille (100) selon la revendication 3, dans laquelle la seconde partie d'aiguille (123) est formée selon une forme de sorte que sa largeur diminue progressivement de la première extrémité à sa seconde extrémité, la première extrémité étant supérieure à la première partie d'aiguille (122) en largeur afin d'avoir un ardillon (123a) formé au niveau cette dernière.

5. Micro-aiguille (100) selon la revendication 4, dans laquelle lorsque la partie de substrat (110) qui a été en contact avec la peau, est retirée de la peau, l'ardillon (123a) s'accroche dans le tissu sous-cutané de sorte que les première et seconde parties d'aiguille (122, 123) sont encastrées dans le tissu sous-cutané.

6. Micro-aiguille (100) selon la revendication 3, dans laquelle la partie perforée (130) comprend :
plusieurs perforations (131) prévues au niveau de la première extrémité de la première partie d'aiguille (122) pour être agencées le long d'une direction transversale de la première partie d'aiguille (122) à intervalles prédéterminés.

7. Micro-aiguille (100) selon la revendication 6, dans laquelle la partie perforée (130) comprend en outre :
une encoche (132), dans laquelle :
l'encoche (132) est formée au niveau de la première extrémité de la première partie d'aiguille (122) pour être positionnée au niveau des coins opposés de la première partie d'aiguille (122) dans la direction de sa largeur, les encoches (132) respectives communiquant avec la découpe d'aiguille (121).

8. Micro-aiguille (100) selon la revendication 3, dans laquelle l'une ou l'autre parmi la première partie d'aiguille (122) et la seconde partie d'aiguille (123) est prévue avec plusieurs évidements de support de médicament (124) formés sur sa surface périphérique, pour être agencés le long de la surface périphérique de l'une ou l'autre parmi la première partie d'aiguille (122) et la seconde partie d'aiguille (123).

9. Micro-aiguille (100) selon la revendication 8, dans laquelle les évidements de support de médicament (124) sont en outre prévus sur l'une ou l'autre parmi une surface supérieure et une surface inférieure de l'une ou l'autre parmi la première partie d'aiguille (122) et la seconde partie d'aiguille (123).

10. Micro-aiguille (100) selon l'une quelconque des revendications 1 à 9, dans laquelle le métal bioabsorbable comprend au moins l'un parmi le magnésium, le calcium, le zinc et le fer.
